# EUROPEAN PATENT APPLICATION

(11) **EP 4 162 961 A1**
(43) Date of publication of application: **12.04.2023**
(21) Application number: 21306417.3
(22) Date of filing: 08.10.2021
(51) Int. Cl.: A61M 5/19, A61M 5/24, A61M 5/28, A61M 5/31, A61M 5/315, A61M 39/22, A61M 39/00

(54) **ACTIVABLE INJECTION DEVICE**

(71) Applicant: Eveon, 38330 Montbonnot-Saint-Martin (FR)
(72) Inventor: DELOBELLE, Vincent, 38330 Montbonnot-Saint-Martin (FR); OUDOIRE, Patrick, 38330 Montbonnot-Saint-Martin (FR)
(74) Representative: Icosa

(57) **Abstract**

Activable injection device (10) comprising:
- an external casing (14) comprising a first opening to be put in fluidic communication with a first reservoir and a second opening to be put in fluidic communication with a second reservoir
- an internal body comprising a first and a second fluidic paths, being linearly mobile inside the external casing and being thus able to be translated between a fluid gathering position and a fluid injection position
- an injection needle (18) in fluidic communication with the second fluidic path.

The fluid gathering position puts the first opening of the external casing in fluidic communication with the second opening of the external casing through the first fluidic path. The fluid injection position puts one of the openings of the external casing in fluidic communication with the injection needle through the second fluidic path.

## Description

### FIELD OF INVENTION

The present invention relates to an activable injection device, more particularly an injection device easy to use in emergency situations or by patients with few strengths of little technical knowledge or by patient needing to execute complexes drug preparation at home.

### BACKGROUND OF INVENTION

Extemporaneous drug preparation requires to follow specific guidance, using dedicated materials and sometimes to be performed in controlled environment. However, in order to improve patient compliance to the treatment but also in order to improve its living quality without reducing safety, it is necessary to develop new technology which enables to facilitate drug reconstitution.

For such homecare application or potential self-preparation and administration the preparation of the drug necessitates to be easily and safely prepared with a reduced number of steps compared to standard hospital procedures. In order to enable a safe quick and easy injection by some person not specialized in injecting drugs, the use of a very handy injector or activable injection device is preferable.

In the case of emergency use, such a device has to remain within reach of the patient in all circumstances and therefore be compact and easily transportable despite the complexity of the reconstitution process and involved mechanisms. The injector has further to be affordable, reliable and easy to use for any kind of patient even for disabled patients.

In the case of homecare drug reconstitution platform with multiple reconstitution step prior injection, it is necessary to optimize user scenario and minimize patient involvement.

Such devices are required to be able to integrate many functions of the mechanical, fluidic, HMI and safety-of-use type.

To ensure compactness, and minimize user actions over the device, it is necessary to be able to combine several functions to save space, by combining or coordinating at least two functions on the same mechanical/technical component.

### SUMMARY

This invention thus relates to an activable injection device aimed at being connected to at least two fluidic reservoirs, said injection device comprising:
- an external casing comprising at least a first opening aimed at being put in fluidic communication with a first fluidic reservoir and at least a second opening aimed at being put in fluidic communication with a second fluidic reservoir,
- an internal body comprising a first fluidic path and a second fluidic path, the internal body being linearly mobile inside the external casing and being thus able to be translated between a fluid gathering position and a fluid injection position,
- an injection needle being in fluidic communication with the second fluidic path of the internal body,

**wherein** the fluid gathering position of the internal body puts the first opening of the external casing in fluidic communication with the second opening of the external casing by means of the first fluidic path,
**wherein** the fluid injection position of the internal body puts one of the openings of the external casing in fluidic communication with the injection needle by means of the second fluidic path.

Thus, this solution achieves the above objective. In particular, it makes it possible to reduce the volume, thus improving compactness and further reducing the costs of activable device for mixing and/or reconstituting medicines. Compactness is improved by the association of the following two linearly activated functions:
- a fluidic switching by translation (linear distributor function), and
- an establishment of a fluidic connection with the needle.

In some preferred embodiment, the linearly mobile internal body inside the external casing is able to be translated between a fluid gathering position, at least one fluid mixing position, and a fluid injection position.

In this embodiment, compactness is improved by the association of the following two linearly activated functions:
- a fluidic switching by translation (linear distributor function), and
- the translational movement of the needle.

Both elements are set into motion by the action of a single actuator and thus a single actuation by a user, rendering the whole drug preparation and injection process very safe and easy to use for any kind of persons, even persons not used to the handling of medical devices.

The device according to the invention may include one or more of the following characteristics, taken in isolation from one another or in combination with one another:
- the internal body, when put in its fluid gathering position, is entirely comprised within the external casing,
- the internal body, when put in its fluid injection position, extends at least partially outside the external casing,
- the injection needle is translated from a resting position to an injection position in a synchronized way with the translation of the internal body from its fluid gathering position to its fluid injection position,
- the injection needle is secured to the internal body,
- the injection needle is secured to the external casing,
- the device further includes an actuator aimed at least at moving the internal body between its fluid gathering position and its injection position,
- the actuator is connected to the internal body by means of an actuator link,
- the actuator is a mechanically activable actuator,
- actuator comprises an activation motor,
- the actuator is activated by means of a switch or a button.

A further object of the present invention regards an injection method implemented by means of an activable injection device according to any one of the here-above listed technical features, the injection method comprising the following steps in the order of enunciation:
- putting the injection device in its fluid gathering position and the needle in its resting position,
- connecting the first opening to a first fluidic reservoir,
- connecting the second opening to a second fluidic reservoir,
- circulating, through the first fluidic path, fluid from one fluidic reservoir, thus obtaining a mixed fluid,
- translating the internal body from its fluid gathering position to its injection position and the injection needle from its resting position to its injection position,
- circulating the mixed fluid from the remaining filled fluidic reservoir to the injection needle through the second fluidic path of the internal body.

The injection method according to the present invention may further include further step: the fluid from the remaining filled reservoir is circulated several times from one fluidic reservoir to another before obtention of the desired mixed fluid and before translating the internal body from its fluid gathering position to its injection position.

### BRIEF DESCRIPTION OF THE DRAWINGS

The invention will be better understood, and other aims, details, characteristics and advantages thereof will emerge more clearly on reading the detailed explanatory description which follows, of embodiments of the invention given by way of illustration. purely illustrative and non-limiting examples, with reference to the accompanying drawings.
- **Figures 1a and 1b** are schematic global perspective views of a first embodiment of the present invention,
- **Figure 2** is an open view of the inside of an external casing of the device according to the present invention,
- **Figures 3a** **and** **3b** are two schematic views of the two positions of the internal body of the device according to the present invention,
- **Figures 4a and 4b** are two schematic side open views of the device according to the present invention in which the needle and the internal bodies positions are depicted with regards to a patient's skin part,
- **Figures 5a** **and** **5b** are two schematic views of an alternative embodiment of the present invention,
- **Figures 6a, 6b, 6c and 6d** are a schematical side view of a further alternative embodiment of the present invention in three different positions,
- **Figure 7** is a perspective view of a third embodiment of the present invention,
- **Figures 8a** **and** **8b** are transparent views of the embodiment of figure 7 in the two positions
- **Figure 9** is a perspective view of a fourth embodiment of the present invention,
- **Figures 10a, 10b, 10c and 10c** are schematics depictions of the different positions of the embodiment of figure 9.

### DETAILED DESCRIPTION

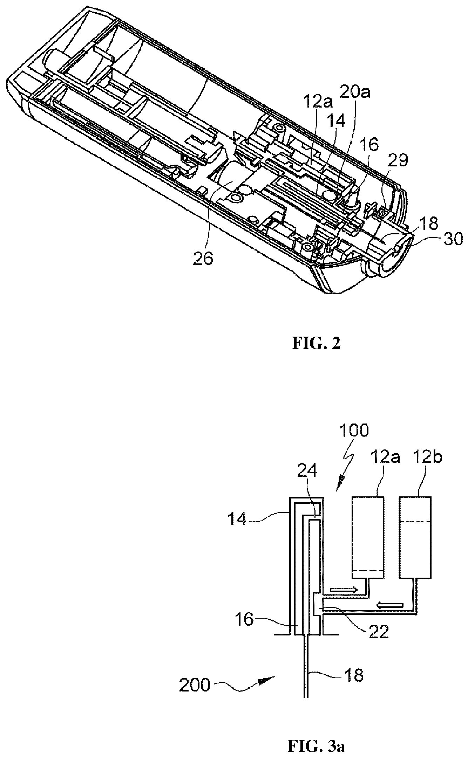

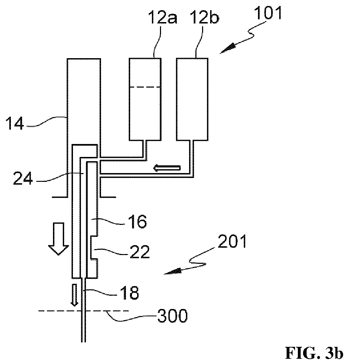

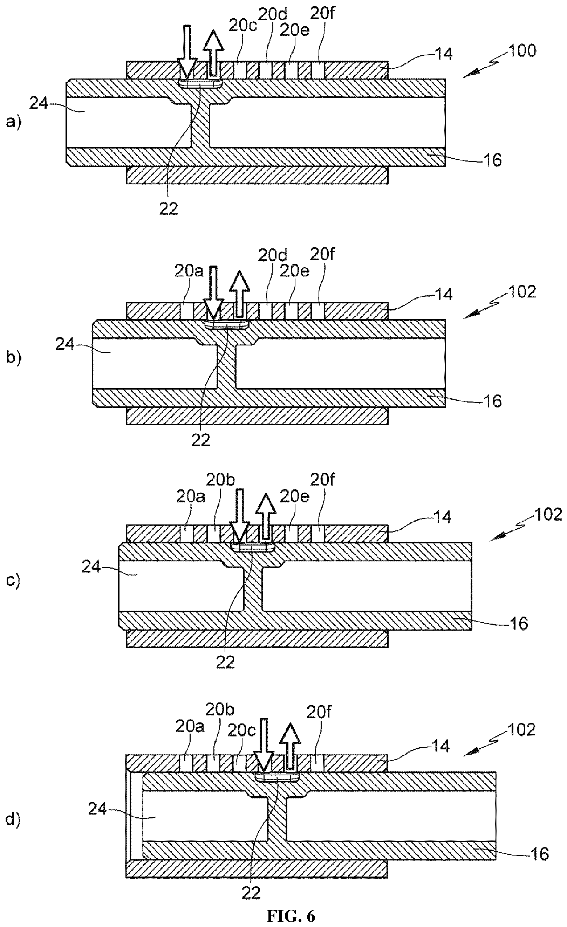

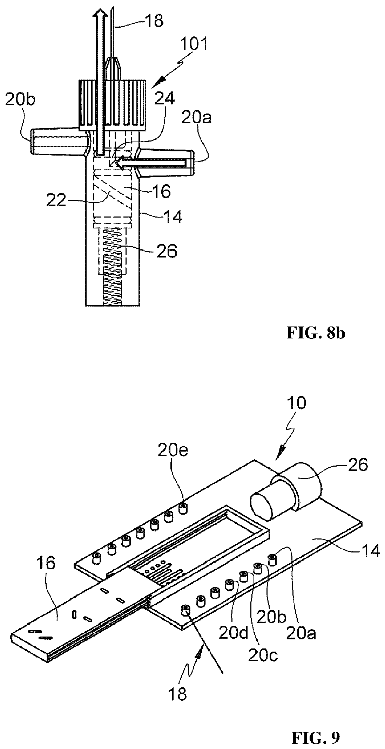

As can be seen on figure 2, 3a and 3b, the activable injection device 10 according to the present invention is aimed at being connected to at least two fluidic reservoirs, a first fluidic reservoir 12a, and a second reservoir 12b. In some embodiments, for example depicted on figures 6a, 6b, 6c and 6d, the injection device 10 can be connected up to four fluidic reservoirs and in the embodiment of figure 9, there is room for up to thirteen reservoirs.

The injection device 10 comprises an external casing 14, an internal body 16 and an injection needle 18.

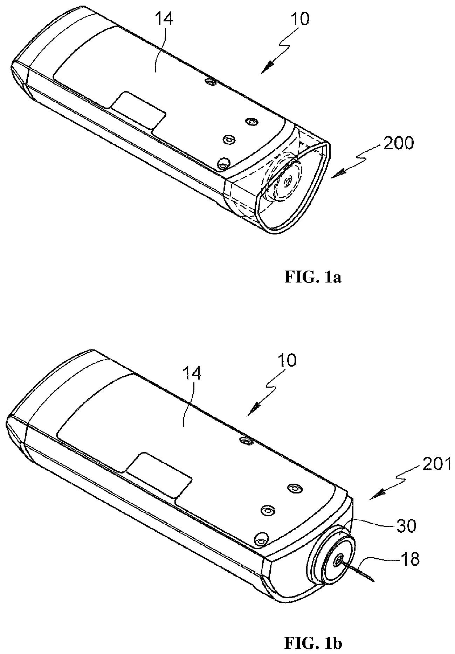

The external casing 14 might be big enough to actually comprise one or more internal compartments 14a, 14b aimed at comprising the at least two fluidic reservoirs 12a, 12b (see figure 3). As can be seen on figures 1a and 1b, the external casing 14 may further display a size and shape which is handy and easy to grab for a user's hand, for example a patient or an operator.

The external casing 14 comprises at least a first opening 20a aimed at being put in fluidic communication with the first fluidic reservoir 12a and at least a second opening 20b aimed at being put in fluidic communication with the second fluidic reservoir 12b. As can be seen on the embodiments of figures 6a, 6b, 6c, 6d and 9, the number of openings 20c, 20d, 20e, 20f, ... can be much higher, depending on the number of fluidic reservoirs 12a, 12b.

The fluid tightness within the injection device 10 is done by the contact between the hard surfaces of the internal body 16 and those of the external body 14 in contact with the said internal body 16. In some embodiments, silicone oil can be used between the internal body 16 and the external casing 14 to improve fluid tightness.

The internal body 16 comprises a first fluidic path 22 and a second fluidic path 24. The injection needle 18 is in fluidic communication with the second fluidic path 24 of the internal body 16. In some embodiments, for example depicted on figure 9, the internal body 16 comprises up to four fluidic paths 220, 221, 222, 24.

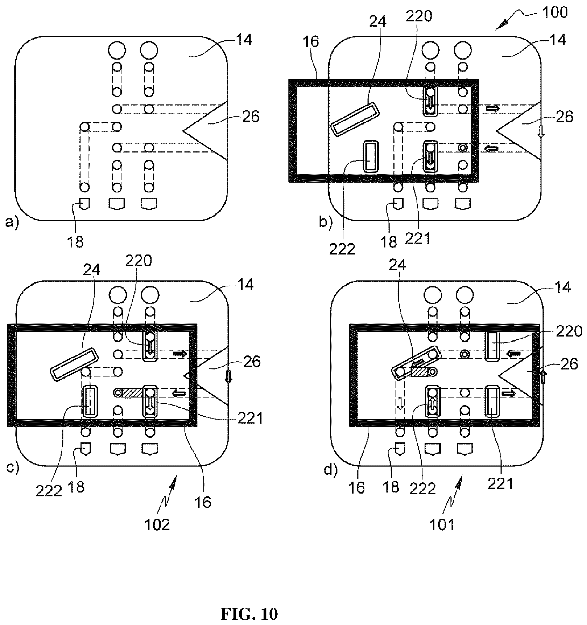

Regardless of the embodiment, the internal body 16 is linearly mobile inside the external casing 14. Regarding the present invention, a linear movement is defined as a general linear movement. This general linear movement can include some rotational or sinusoidal components. The internal body 16 is thus able to be translated, inside the external casing 14, between a fluid gathering position 100 (see figures 3a, 6a, 6b, 6c, 8a, 10b, 10c) and a fluid injection position 101 (see figures 3b, 6c, 8b, 10d).

As can be seen on the embodiment depicted on figure 3a, when the internal body 16 is put in its fluid gathering position 100, the internal body 16 is entirely comprised within the external casing 14 which enables a total protection and preserve sterility prior activation. This also improves device compactness. More generally speaking and regardless of being entirely comprised or not inside the external casing, as can be seen on figures 6a, 6b, 6c, 8a, 10b, 10c, the fluid gathering position 100 of the internal body 16 puts the first opening 20a of the external casing 14 in fluidic communication with the second opening 20b of the external casing 14 by means of the first fluidic path 22, 220.

On the other hand, as can be seen on figure 3b, in some embodiments, when the internal body 16 is put in its fluid injection position 101, the internal body 16 extends at least partially outside the external casing 14. Organizing the different fluidic path according linearity of the internal body enables an easier special arrangement. It also enables to use the linear movement of the internal body as a mean for injection, thus reducing the potential number or actuators within the device. More generally speaking and regardless of being entirely comprised or not inside the external casing, as can be seen on figures 3b, 6d, 8b, 10d, the fluid injection position 101 of the internal body 16 puts one of the openings 20a, 20b, 20c, 20d, 20e, 20f, preferably the opening associated to the remaining filled fluidic reservoir 12a, 12b of the external casing 14, in fluidic communication with the injection needle 18 by means of the second (or fourth) fluidic path 24.

In some embodiments, the internal body 16 is linearly mobile inside the external casing 14 and able to be translated between a fluid gathering position 100, a fluid mixing position 102, and at least one fluid injection position 101. Each fluid mixing position 102 is an intermediate position in which different openings 20a, 20b, 20c, 20d, 20e, 20f are put in fluidic connection by one or several fluidic paths 22, 220, 221, 222 in order to move the fluid comprised in the first fluidic reservoir 12a, to the second or further fluidic reservoirs 12b, in order to add more reactants to the final mix or simply improve the overall mixing of the reactants already put together. This enables a standardized reconstitution and potential additional step (dilution or dosing). Such device enables a standardized drug preparation as well as a reduction of drug preparation procedure when using multiple containers. It also enables standardization in any possible conditions, which includes emergency use of the device.

In order to translate the internal body 16 from its fluid gathering position 100 to its fluid injection position 101, the device 10 may further include an actuator 26 (see figures 2, 8 and 9) aimed at least at moving the internal body 16 between its fluid gathering position 100 and its fluid injection position 101. In case the internal body 16 also comprises one or several fluid mixing position(s) 102, the actuator 26 also enables to move the internal body 16 between the different positions 100, 101, 102, including the fluid gathering position 100, each fluid mixing position 102 and the fluid injection position 101.

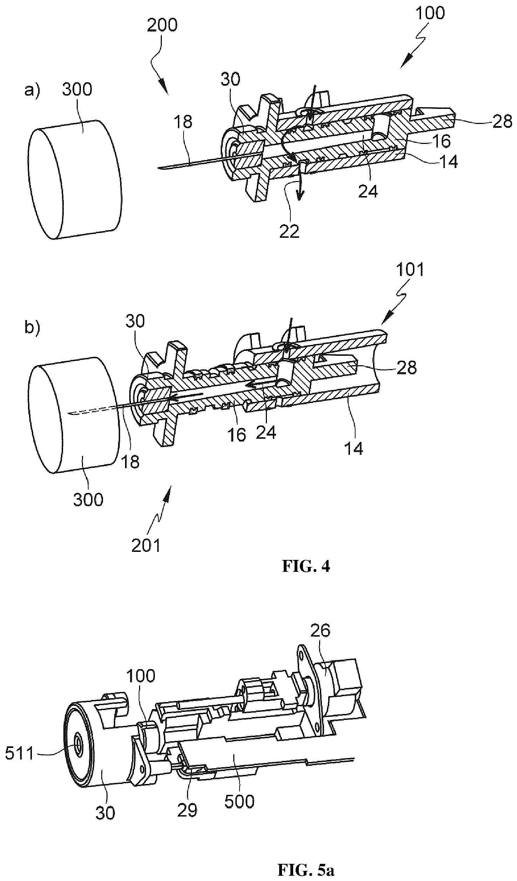

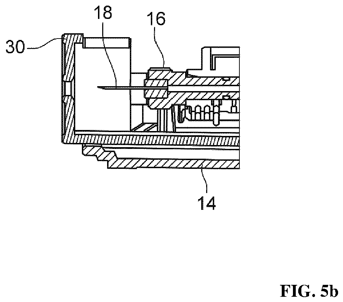

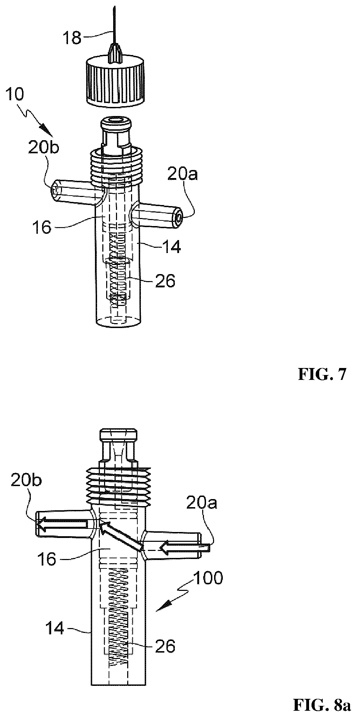

As can be deduced from figures 2, 4a, 4b, in some embodiments, the actuator 26 is connected to the internal body 16 by means of an actuator link 28. This actuator link 28 may be a permanent or retrievable mechanical coupling such as harpooning clip. The actuator link 28 might for example be situated at the front of the internal body 16 (see figures 5a and 5b) or at the back of the internal body 16 (see figure 8a, 8b)

The technical usefulness of the actuator 26 is to allow automated or mechanical control of the medicine preparation and the needle descent. More precisely, the actuator 26 ensures that the needle 18 only descents:
- once the preparation of the medicine has been completed,
- when the injection device 10 is well positioned on the patient.

The actuator 26 may enable the patient to decide when to trigger the injection and adds thus to the comfort and safety felt by the patient who is in control (in case the patient is doing the injection). The mechanical or automated control of the descent of the needle 18 (which is not controlled directly by the patient or and operator), allows a better and more precise control of the injection time, which improves safety and injection precision.

The use of an actuator 26 also allows the injection to take place without the patient having to see/watch the needle 18, which has the effect of reducing patient stress.

In some embodiments, the safety and precision of the descent of the needle 18 can be improved by the addition of a detector (not represented) ensuring that the injection device 10 is in good contact with the patient.

In some embodiments (see figure 7, 8a, 8b), the actuator 26 is a purely mechanical actuator activated by means of an operator's action, without any electronical input. In those cases, the actuator 26 may for example comprise a mechanically activable activation jack. In an alternative embodiment, the actuator 26 may comprise a mechanically activable activation spring. The actuator may also been set in motion using compressed air.

In some other embodiments (see figure 9), the actuator 26 is an electromechanical actuator and comprises an activation motor or an electromagnet. In some embodiments, the activation motor is for example a linear motor or, alternatively, a rotary motor which drives a helical rod integrated into the internal body 16 and converts the rotation of the activation motor into translation for the internal body 16 and descent of the needle 18. In some alternative embodiment, the translation of the internal body 16 and the needle 18 are obtained by a bistable or monostable hydraulic control (preferably with a return spring).

When the actuator 26 is a motor or an electromagnet, a device activation system can be used to induce the translation of the internal body 16 inside the external casing 14. In those embodiments, the actuator 26 is activated for example by means of an electronical switch or a button. More precisely, the device activation system may be composed of:
- an electronic card with an electrical switch 29 (see figure 5a),
- a pricking button 30 (see figures 5a and 5b).

When the electrical switch 28 is pressed, the actuator 26 is powered and translates automatically the internal body 16 inside the external casing 14. The pricking button 30 can be used to press on the electrical switch 29. In those embodiments, the pricking button 30 also protect the user from unintentional pricking with the needle 18 as the pricking button 30 does not allow the access to the needle 18 when pressed. The use of such a pricking button 30 simplifies the use of the injection device 10 and reduce the number of usage steps: the pricking button 30 can be directly applied on the skin 300 of the patient and can trigger the actuator 26 when the injection device 10 is pushed against said skin 300. Thus, the pricking button 30 is preferably secured to the front surface of the external casing 14. A hole 31 on the pricking button 30 is a possible way for the needle 18 to descent. The stroke of the needle 18 can be adjusted with the stroke of the pricking button 30 in order to have a pricking depth of the needle 18 corresponding to subcutaneous or intramuscular injection.

The presence of a needle 18 comprised in the injection device 10 makes it possible to avoid the implementation of a further fluidic connection (usually flexible) between the needle 18 and the external casing 14 which avoids all sorts of sterility problems. Both elements are set into motion by the action of a single actuator: this enables to reduce the number of component and improve compactness of the device.

The needle 18 can be either directly assembled at the distal extremity of the internal body 16, for high external diameter needles, or alternatively assembled into a needle socket assembled to the internal body 16, for small external diameter needles. More precisely, in some embodiments, the injection needle 18 is secured to the internal body 16 (see figures 2, 3b and 4b). This way, the injection needle 18 is translated from a resting position 200 to an injection position 201 in a synchronized way with the translation of the internal body 16 from its fluid gathering position 100 to its fluid injection position 101. In some alternative embodiments (see figures 8 and 9), the injection needle 18 is not secured to the internal body 16, but secured to the external casing 14. However, even in those embodiments, the injection needle 18 is translated from a resting position 200 to an injection position 201 in a synchronized way with the translation of the internal body 16 from its fluid gathering position 100 to its fluid injection position 101. This can be achieved by a temporary connection between the injection needle 18 and the internal body 16 or by a synchronized reaction chain moving both elements independently one from the other but nevertheless in a synchronized way.

An alternative to the mechanical or automatic descent of the needle 18 could be the masking of the needle 18 by an elastic sheath which retracts when the patients apply the injection device 10 against their skin 300. As a result, the needle 18 remains invisible during the operation.

While activating of the actuator 26 and an automated or mechanically induced and controlled preparation operation, the user, preferably the patient, can thus easily apply the injecting device against the patient's skin 300 and then voluntarily initiates or wait for the injection. Preferably, the activation of the actuator 26 induces both the drug preparation operation and the injection, reducing the number of step of the user scenario and limiting a complex task to a limited number of action.

In some embodiments, the internal body 16 and the injection needle 18 are discardable. This might reduce problems due to disinfection. This is the case for example in the embodiment of figure 8 where part of the external casing 14 is also discardable.

The here-above presented activable injection device 10 enables the implementation of an injection method, said injection method comprising the following steps in the order of enunciation:
- putting the injection device 10 in its fluid gathering position 100 and the needle 18 in its resting position 200,
- connecting the first opening 20a to a first fluidic reservoir 12a, and preferably simultaneously the second opening 12b to a second fluidic reservoir 20b,
- circulating, through the first fluidic path 22, 220, fluid from one reservoir to the other, thus obtaining a mixed fluid,
- translating the internal body 16 from its fluid gathering position 100 to its injection position 101 and the injection needle 18 from its resting position 200 to its injection position 201,
- circulating mixed fluid from the filled fluidic reservoir 20a, 20b to the injection needle 18 through the second fluidic path 24 of the internal body 16.

The fluid from the second reservoir is circulated several times from the second reservoir to the first reservoir and from the first reservoir to the second reservoir before obtention of the mixed fluid stored in the filled reservoir before translating the internal body 16 from its fluid gathering position to its injection position.

The internal cavity 16 might be translating from the fluid gathering position 100 to one or several fluid mixing positions 102 before being finally translated to its fluid injection position 101. Each fluid mixing position 102 may enable to connect more fluid reservoirs to the filled reservoir in order to improve mixing or add new reactants.

The here-above presented activable injection device 10 further enables the implementation of an alternative drug preparation method, said injection method comprising the following steps in the order of enunciation:
- putting the injection device 10 in its fluid gathering position 100 and the needle 18 in its resting position 200,
- translating the internal body 16 from its fluid gathering position 100 to its fluid injection position 101 in order to suck or sample a fluid, and the injection needle 18 from its resting position 200 to its injection position 201, to fill a fluidic reservoir 12a,
- putting the injection device 10 in its fluid gathering position 100 and the needle 18 in its resting position 200,
- connecting the first opening 20a to a first fluidic reservoir 12a, and preferably simultaneously the second opening 12b to a second fluidic reservoir 20b,
- circulating, through the first fluidic path 22, 220, fluid from one reservoir to the other, thus obtaining a mixed fluid,
- translating the internal body 16 from its fluid gathering position 100 to its injection position 101 and the injection needle 18 from its resting position 200 to its injection position 201,
- circulating mixed fluid from the filled fluidic reservoir 20a, 20b to the injection needle 18 through the second fluidic path 24 of the internal body 16.

The fluid from the second reservoir is circulated several times from the second reservoir to the first reservoir and from the first reservoir to the second reservoir before obtention of the mixed fluid stored in the filled reservoir before translating the internal body 16 from its fluid gathering position to its injection position.

The internal cavity 16 might be translating from the fluid gathering position 100 to one or several fluid mixing positions 102 before being finally translated to its fluid injection position 101. Each fluid mixing position 102 may enable to connect more fluid reservoirs to the filled reservoir in order to improve mixing or add new reactants.

## Claims

1. Activable injection device (10) aimed at being connected to at least two fluidic reservoirs (12a, 12b), said injection device (10) comprising:
- an external casing (14) comprising at least a first opening (20a) aimed at being put in fluidic communication with a first fluidic reservoir (12a) and at least a second opening (20b) aimed at being put in fluidic communication with a second fluidic reservoir (12b),
- an internal body (16) comprising a first fluidic path (22) and a second fluidic path (24), the internal body (16) being linearly mobile inside the external casing (14) and being thus able to be translated between a fluid gathering position (100) and a fluid injection position (101),
- an injection needle (18) being in fluidic communication with the second fluidic path (24) of the internal body (16),
**wherein** the fluid gathering position (100) of the internal body puts the first opening (20a) of the external casing (14) in fluidic communication with the second opening (20b) of the external casing (14) by means of the first fluidic path (22),
**wherein** the fluid injection position (101) of the internal body (16) puts one of the openings (20a, 20b) of the external casing (14) in fluidic communication with the injection needle (18) by means of the second fluidic path (24).

2. Activable injection device (10) according to the preceding claim, wherein the linearly mobile internal body (16) inside the external casing (14) is able to be translated between a fluid gathering position (100), at least one fluid mixing position (102), and a fluid injection position (101).

3. Activable injection device (10) according to the precedent claim, wherein the internal body (16), when put in its fluid gathering position (100), is entirely comprised within the external casing (14).

4. Activable injection device (10) according to any one of the preceding claims, wherein the internal body (16), when put in its fluid injection position, (101) extends at least partially outside the external casing (14).

5. Activable injection device (10) according to the preceding claim, wherein the injection needle (18) is translated from a resting position (200) to an injection position (201) in a synchronized way with the translation of the internal body (16) from its fluid gathering position (100) to its fluid injection position (101).

6. Activable injection device (10) according to the preceding claim, wherein the injection needle (18) is secured to the internal body (16).

7. Activable injection device (10) according to claim 5, wherein the injection needle (18) is secured to the external casing (14).

8. Activable injection device (10) according to any one of the preceding claims, wherein the device (10) further includes an actuator (26) aimed at least at moving the internal body (16) between its fluid gathering position (100) and its injection position (101).

9. Activable injection device (10) according to the preceding claim, wherein the actuator (26) is connected to the internal body (16) by means of an actuator link (28).

10. Activable injection device (10) according to claims 8 or 9, wherein the actuator (26) is a mechanically activable actuator (26).

11. Activable injection device (10) according to claims 8 or 9, wherein the actuator (26) comprises an activation motor.

12. Activable injection device (10) according to any one of claims 7 to 10, wherein the actuator (26) is activated by means of a switch (29) or a button (30).

13. Injection method implemented by means of an activable injection device (10) according to any one of claims 1 to 13, the injection method comprising the following steps in the order of enunciation:
- putting the injection device (10) in its fluid gathering position (100) and the needle (18) in its resting position (200),
- connecting the first opening (20a) to a first fluidic reservoir (12a),
- connecting the second opening (20b) to a second fluidic reservoir (12b),
- circulating, through the first fluidic path (22), fluid from one fluidic reservoir (12a, 12b), thus obtaining a mixed fluid,
- translating the internal body (16) from its fluid gathering position (100) to its injection position (101) and the injection needle (18) from its resting position (200) to its injection position (201),
- circulating the mixed fluid from the remaining filled fluidic reservoir (12a, 12b) to the injection needle (18) through the second fluidic path (24) of the internal body (16).

14. Injection method according to the preceding claim, wherein the fluid from the remaining filled reservoir (12a, 12b) is circulated several times from one fluidic reservoir to another before obtention of the desired mixed fluid and before translating the internal body (16) from its fluid gathering position (100) to its injection position (101).
